# FASCICULE DE BREVET EUROPEEN

(11) **EP 0 983 777 B1**
(45) Date de publication et mention de la délivrance du brevet: **24.03.2004**
(21) Numéro de dépôt: 99402095.6
(22) Date de dépôt: 20.08.1999
(51) Int. Cl.: A62D 3/00, C07C 209/84

(54) **Procédé de traitement de fruits et légumes après récolte avec purification de produits phytosanitaires contaminés par des amines primaires aromatiques**
Verfahren zur Behandlung von Früchten und Gemüse nach der Ernte unter Reinigung von mit primären aromatischen Aminen kontaminierten phytosänitaren Produkten
Process for the post-harvest treatment of fruits and vegetables with purification of phytosanitary products contaminated with primary aromatic amines

(30) Priorité: 02.09.1998 FR 9810980
(43) Date de publication de la demande: 08.03.2000
(73) Titulaire: XEDA INTERNATIONAL, 13670 Saint-Andiol (FR)
(72) Inventeur: Sardo, Alberto, 13160 Chateaurenard (FR)
(74) Mandataire: Bernasconi, Jean Raymond

(56) Documents cités:
- EP-A- 0 084 285
- EP-A- 0 522 891
- FR-A- 2 636 503
- FR-A- 2 689 506
- US-A- 5 709 800
- CHEMICAL ABSTRACTS, vol. 82, no. 21, 26 mai 1975 (1975-05-26) Columbus, Ohio, US; abstract no. 139552, WIEHERT ET AL.: "Nitrosation and condensation of aromatic compounds with sodium nitrite..." XP002100751 & Z. CHEM., vol. 15, no. 2, 1975, pages 49-50,

## Description

La présente invention concerne un procédé simplifié de- purification de produits phytosanitaires contaminés par des impuretés de type amines primaires aromatiques.

De nombreux produits phytosanitaires couramment utilisés dans le traitement des fruits et légumes sont contaminés, sous leur forme commerciale, par des substances toxiques indésirables. C'est le cas notamment de la diphénylamine, laquelle est largement utilisée en raison de ses propriétés antioxydantes, qui est contaminée par divers sous-produits de fabrication, notamment des amines aromatiques primaires telles que l'aniline, le 4-aminobiphényle et le 2-aminobiphényle.

C'est également le cas de l'éthoxyquin, produit antioxydant, lequel est contaminé par la p-phénétidine. On peut de même citer le chlorprofam qui est un antigerminatif de la pomme de terre et qui contient des quantités non négligeables de métachloroaniline.

FR 2 689 506 décrit un procédé assurant l'élimination sélective des amines primaires contaminant la diphénylamine par mise en contact d'une solution de diphénylamine contaminée avec une résine échangeuse d'ions cationique en présence d'eau. Ce procédé quoique efficace est complexe et laborieux. Il nécessite soit l'utilisation de colonnes garnies de résine échangeuse d'ions, soit la séparation de la résine échangeuse d'ions du milieu réactionnel avant traitement des fruits et légumes.

Le procédé de l'invention ne présente pas ces inconvénients. Selon l'invention, en effet, le milieu réactionnel récupéré à l'issue du procédé est directement utilisable pour la préparation d'une composition phytosanitaire destinée au traitement des fruits et légumes. La mise en oeuvre du procédé de l'invention est en outre particulièrement simple et ne nécessite pas un appareillage sophistiqué, ni coûteux.

Le procédé revendiqué vise à débarrasser un produit phytosanitaire donné de ses impuretés de type amine primaire aromatique. Le procédé de l'invention est efficace quelle que soit la nature exacte et la quantité desdites impuretés de type aromatique. Il est particulièrement approprié à l'élimination des impuretés de formule I :

Ar-NH₂ I

dans laquelle Ar représente un noyau aromatique monocyclique ou polycyclique éventuellement substitué par un ou plusieurs radicaux choisis parmi un atome d'halogène; un groupe (C₁-C₈)alkyle, (C₁-C₈)alcoxy; et amino.

On entend par groupe alkyle, une chaîne hydrocarbonée linéaire ou ramifiée. De préférence, ledit groupe alkyle comprend de 1 à 5 atomes de carbone.

La partie alkyle du groupe alcoxy est définie de façon analogue.

De manière particulièrement avantageuse, le noyau aromatique Ar est mono- ou bicylique. A titre d'exemples, on peut citer les groupes phényle, naphtyle, anthryle et phénanthryle, les groupes phényle et naphtyle étant préférés.

Le procédé de l'invention est plus particulièrement approprié à la purification de produits phytosanitaires comprenant jusqu'à 10 000 ppm en poids d'impuretés de type amine primaire aromatique, de préférence jusqu'à 1000 ppm.

Le procédé de l'invention consiste à traiter, en milieu acide, le produit phytosanitaire avec un nitrite de métal alcalin, tel que le nitrite de sodium ou le nitrite de potassium.

Plus précisément, le procédé de l'invention comprend:
a) la mise en contact, sous agitation à une température comprise entre 15 et 25°C, d'une solution aqueuse dudit nitrite de métal alcalin avec une solution organique préparée par dissolution dudit produit phytosanitaire à purifier dans un solvant choisi parmi un tensioactif non-ionique, un glycol en C₂-C₁₂ et leurs mélanges;
b) l'addition au milieu réactionnel résultant, maintenu sous agitation à ladite température, d'un acide minéral fort;
c) puis, le chauffage du milieu réactionnel à une température comprise entre 30 et 70°C.

La première et la deuxième étapes du procédé revendiqué sont généralement réalisées dans l'ordre indiqué. Toutefois, il est possible, dans le cadre de l'invention, d'acidifier dans un premier temps la solution aqueuse du nitrite de métal alcalin, puis de mettre en contact sous agitation ladite solution aqueuse acidifiée avec la solution organique du produit phytosanitaire, avant de chauffer le milieu réactionnel à une température comprise entre 30 et 70°C.

Il doit être entendu cependant que le mode de réalisation consistant à effectuer les étapes a) et b) dans l'ordre indiqué (étape a) puis étape b)) est préféré.

La solution aqueuse du nitrite de métal alcalin est de préférence une solution aqueuse contenant 0,5 à 3 mol/litre de nitrite de métal alcalin, mieux encore de 0,5 à 1,5 mol/litre, par exemple une solution molaire.

La quantité de ladite solution aqueuse mise en oeuvre à l'étape a) dépend de la quantité d'impuretés de type amine aromatique primaire contenue dans la solution organique du produit phytosanitaire.

En toute rigueur, une quantité stoechiométrique du nitrite de métal alcalin par rapport aux moles totales de groupes amino primaire aromatique présentes dans la solution organique suffit pour une élimination complète des impuretés. On notera qu'une impureté donnée peut contenir plus d'un groupe amino primaire aromatique. C'est le cas, notamment, des impureté de formule Ar-NH₂, dans laquelle Ar représente un noyau aromatique substitué par plus d'un groupe amino.

Toutefois, il est souhaitable d'utiliser un excès du nitrite de métal alcalin. Une quantité variant entre 1 et 5 équivalents molaires du nitrite de métal alcalin par rapport aux moles totales de groupes amino primaire aromatique, par exemple entre 1 et 3 équivalents molaires, est appropriée.

La solution organique du produit phytosanitaire est préparée par dissolution du produit phytosanitaire dans un solvant qui est choisi parmi un tensioactif non-ionique, un glycol en C₂-C₁₂ et leurs mélanges dans des proportions quelconques.

Selon l'invention on préfère néanmoins que ledit solvant soit constitué :
- d'un ou plusieurs tensioactifs non-ioniques;
- ou bien d'un mélange d'un ou plusieurs tensioactifs non-ioniques avec un ou plusieurs glycols en C₂-C₁₂.

De façon particulièrement avantageuse, ledit solvant est constitué d'un ou plusieurs tensioactifs non-ioniques.

Par glycols, on entend dans le cadre de l'invention, les alcools dihydroxylés dérivés des hydrocarbures aliphatiques par remplacement de deux atomes d'hydrogène avec deux groupes hydroxyles.

Selon l'invention, on préfère les glycols en C₂-C₆, et notamment l'éthylèneglycol et le propylèneglycol.

Des exemples de tensioactifs non-ioniques utilisables selon l'invention son notamment :
- le produit de condensation d'un alcool gras aliphatique, de préférence en C₈-C₂₂, avec un oxyde d'alkylène en C₂-C₃. L'oxyde d'alkylène en C₂-C₃ peut être l'oxyde d'éthylène, l'oxyde de propylène, ou bien un mélange d'oxyde d'éthylène et d'oxyde de propylène dans des proportions quelconques. Un exemple de tels tensioactifs est le produit de condensation de l'alcool laurylique (ou alcool n-dodécylique) avec 30 moles d'oxyde d'éthylène;
- le produit de condensation d'un alkylphénol dans lequel la chaîne alkyle est en C₈-C₂₂ avec un oxyde d'alkylène en C₂-C₃. Là encore, les produits de condensation avec l'oxyde d'éthylène, l'oxyde de propylène ou bien un mélange d'oxyde d'éthylène et d'oxyde de propylène dans des proportions quelconques sont également avantageux. A titre d'exemple de tels tensioactifs, on peut citer le produit de condensation du n-nonylphénol avec 10 moles d'oxyde d'éthylène;
- le produit de condensation d'un acide gras de préférence en C₈-C₂₂ avec un oxyde d'alkylène en C₂-C₃, par exemple l'oxyde d'éthylène ou l'oxyde de propylène ou un mélange d'oxyde d'éthylène et d'oxyde de propylène dans des proportions quelconques. Ces produits de condensation présentent une chaîne alkoxylée au niveau de la fonction hydroxyle du groupe carboxylique. Des tensioactifs préférés de ce groupe sont les produits de condensation obtenus à partir de l'acide oléique, de l'acide palmitique et de l'acide stéarique.

Habituellement, les tensioactifs utilisés résultent de la condensation d'un alcool gras, d'un acide gras ou d'un alkylphénol avec 3 à 50 moles d'oxyde d'alkylène en C₂-C₃.

Parmi les tensioactifs mentionnés ci-dessus, ceux présentant une constante HLB (équilibre hydrophyle-lipophile) comprise entre 6 et 18 sont particulièrement préférés.

La solution organique mise en oeuvre à l'étape a) comprend de préférence de 5 à 50% en poids dudit produit phytosanitaire, mieux encore de 10 à 30% en poids.

La mise en contact de la solution aqueuse avec la solution organique du produit phytosanitaire est réalisée à une température comprise entre 15 et 25°C, sous agitation.

A l'étape b), l'acide minéral est un acide fort; en tout état de cause un acide plus fort que HNO₂ est souhaitable. Des exemples en sont l'acide sulfurique et l'acide chlorhydrique.

La quantité d'acide fort devant être utilisée est généralement comprise entre 1,5 et 3,5 équivalents molaires par rapport à la quantité de nitrite de métal alcalin mise en jeu; de préférence, on utilise deux équivalents d'acide fort.

L'acide minéral fort peut être ajouté au mélange réactionnel sous forme pure ou sous la forme d'une solution aqueuse diluée. A titre de solution aqueuse diluée, on préfère les solutions de 0,5 à 3 N, mieux encore de 0,5 à 1,5 N, par exemple 1N.

A l'étape c) le mélange réactionnel est maintenu sous agitation et chauffé à une température comprise entre 30 et 70°C, de préférence entre 40 et 50°C, le temps nécessaire pour une élimination complète des impuretés de type amine primaire aromatique.

L'homme du métier pourra par exemple surveiller l'avancement de la réaction en prélevant et analysant, à intervalles réguliers, des aliquotes du milieu réactionnel.

Généralement, la réaction est terminée après 30 minutes à 5 heures, voire 30 minutes à 3 heures.

De façon particulièrement avantageuse, le milieu réactionnel résultant de la mise en oeuvre du procédé de l'invention est directement utilisable, après addition d'eau, pour le traitement de fruits et légumes après récolte. Suivant la nature du produit phytosanitaire présente dans le milieu réactionnel, le traitement aura un effet antioxydant, antigerminatif de la pomme de terre, ou/et fongicide.

Ainsi, selon un autre de ses aspects, l'invention concerne un procédé pour le traitement de fruits et légumes après récolte, caractérisé en ce que l'on utilise, à titre de composition phytosanitaire traitante, le milieu réactionnel résultant de la mise en oeuvre du procédé de l'invention en mélange avec de l'eau.

Une première variante préférée de l'invention consiste à mettre le milieu réactionnel, obtenu par mise en oeuvre du procédé de l'invention, en dispersion dans de l'eau avant utilisation. La dispersion est préparée en utilisant de préférence une quantité d'eau telle que le rapport volumique du milieu réactionnel à l'eau est compris entre 1/100 et 1/1000, de préférence entre 1/50 et 1/1000. Dans ce but, le milieu réactionnel brut peut être stocké et commercialisé tel quel, sous la forme de concentré émulsifiable.

Une seconde variante préférée de l'invention consiste à ajouter au milieu réactionnel jusqu'à 30% en poids d'eau. La composition résultante est particulièrement appropriée au traitement des fruits et légumes par nébulisation. Dans ce cas, la composition phytosanitaire peut être stockée, après addition d'eau au milieu réactionnel, sous la forme de composition nébulisable pour aérosol.

Lorsqu'on envisage d'utiliser le milieu réactionnel pour la préparation d'une composition phytosanitaire pour aérosol, il est avantageux d'utiliser à titre de solvant, à l'étape a), un mélange d'un ou plusieurs tensioactifs non-ioniques et d'un ou plusieurs glycols.

En variante, le solvant étant constitué d'un ou plusieurs tensioactifs non-ioniques, il est conseillé d'incorporer à la solution organique un ou plusieurs co-solvants choisis parmi les alcools aliphatiques en C₁-C₁₂.

Plus généralement, il est possible selon l'invention de préparer la solution organique utilisée à l'étape a) par (i) dissolution du produit phytosanitaire dans un solvant puis (ii) addition d'un ou plusieurs co-solvants choisis parmi les alcools aliphatiques en C₁-C₁₂.

De façon avantageuse, la composition phytosanitaire traitante utilisée pour le traitement des fruits et légumes comprend 5 à 50% en poids du produit phytosanitaire, 0 à 80% en poids de tensioactifs non-ioniques, 0 à 80% en poids de glycols, 0 à 20% en poids d'alcools aliphatiques et 0 à 30% en poids d'eau.

Plus préférablement, ladite composition comprend 5 à 50% en poids du produit phytosanitaire, 5 à 80% en poids de tensioactifs non-ioniques, 0 à 60% en poids de glycols, 0 à 20% en poids d'alcools aliphatiques et 0 à 30% en poids d'eau.

La composition phytosanitaire est obtenue par mise en oeuvre des étapes consistant à :
- préparer une solution aqueuse A d'un nitrite de métal alcalin;
- préparer une solution organique B par dissolution du produit phytosanitaire dans un solvant tel que défini ci-dessus et, le cas échéant, addition d'un ou plusieurs co-solvants (choisis parmi les alcools aliphatiques en C₁-C₁₂);
- mettre en contact la solution A avec la solution B, sous agitation, à une température comprise entre 15 et 25°C;
- additionner au milieu réactionnel résultant, maintenu sous agitation à ladite température, un acide minéral fort;
- chauffer le milieu réactionnel à une température comprise entre 30 et 70°C; et
- ajouter de l'eau au milieu réactionnel résultant.

De façon préférée, la solution organique du produit phytosanitaire mise en oeuvre à l'étape a) comprend donc essentiellement de 5 à 50% en poids dudit produit phytosanitaire; de 0 à 80% en poids, de préférence de 5 à 80% en poids, de tensioactifs non-ioniques; de 0 à 80% en poids, de préférence de 0 à 60% en poids, de glycols en C₂-C₁₂; et de 0 à 20% en poids d'alcools aliphatiques en C₁-C₁₂.

Selon un autre mode de réalisation de l'invention, il est possible d'ajouter à la composition phytosanitaire traitante différents constituants, tels que des cires, en vue de son application sur les fruits et légumes. De tels constituants sont ceux couramment utilisés dans la technique.

L'invention n'entend pas se limiter à la purification d'un seul produit phytosanitaire mais englobe également la purification concomitante de plusieurs produits phytosanitaires dès lors que chacun de ces produits contient des impuretés de type amine primaire aromatique. Dans ce cas, il convient de préparer une solution organique des différents produits phytosanitaires en vue de la mise en oeuvre de l'étape a) du procédé de l'invention.

Le procédé de l'invention est plus particulièrement approprié à la purification :
- de la diphénylamine laquelle est notamment contaminée par l'aniline, le 2-aminobiphényle et le 4-aminobiphényle;
- du chlorprofam lequel est notamment contaminé par la métachloroaniline;
- de l'éthoxyquin laquelle est notamment contaminée par la p-phénétidine.

Dans la suite, l'invention va être décrite en référence aux exemples suivants.

### EXEMPLE 1

### Purification de diphénylamine contaminée par 300 ppm d'aniline et 50 ppm d'un mélange de 2-aminobiphényle et de 4-aminobiphényle

On prépare une solution organique de 200 kg de diphénylamine dans 800 kg d'un mélange des trois tensioactifs non-ioniques suivants :
- le produit de condensation de l'acide oléique avec 6 moles d'oxyde d'éthylène;
- le produit de condensation de l'acide oléique avec 10 moles d'oxyde d'éthylène; et
- le produit de condensation du n-nonylphénol avec 10 moles d'oxyde d'éthylène.

Après dissolution complète de la diphénylamine dans le mélange de tensioactifs, on ajoute à la solution résultante 1 kg d'une solution 1M de nitrite de sodium.

Après une heure d'agitation, on ajoute 2 kg d'une solution 1M d'acide chlorhydrique et on laisse agiter encore 1 heure en chauffant le milieu réactionnel à une température comprise entre 40 et 50°C.

On vérifie par analyse par HPLC (chromatographie liquide haute performance) d'un aliquote du milieu réactionnel que la totalité des contaminants de type amine primaire aromatique a été éliminée.

Le milieu réactionnel est utilisé après dispersion dans de l'eau, en tant que composition traitante dans le traitement de fruits et légumes après récolte. La composition traitante a l'effet antioxydant et fongicide attendu.

### EXEMPLE 2

### Purification de chlorprofam contaminé par 500 ppm de métachloroaniline

On prépare une solution organique de 200 kg de chlorprofam dans 150 kg d'un tensioactif choisi parmi:
- le produit de condensation de l'alcool n-dodécylique avec 30 moles d'oxyde d'éthylène;
- le produit de condensation du n-nonylphénol avec 10 moles d'oxyde d'éthylène; et
- leurs mélanges en des proportions quelconques.

On ajoute à cette solution 650 kg de propylène-glycol.

Après dissolution complète du chlorprofam, on ajoute sous agitation 2kg d'une solution aqueuse 1M de nitrite de sodium. Après une heure d'agitation, on ajoute 4 kg d'une solution aqueuse molaire 1M d'acide chlorhydrique et on laisse agiter encore 1 heure en chauffant le milieu réactionnel à une température comprise entre 40 et 50°C.

On vérifie alors par analyse par HPLC d'un aliquote du milieu réactionnel que la méta-chloroaniline a été éliminée du milieu réactionnel.

Le milieu réactionnel est utilisé, après addition d'eau, sous la forme d'une composition pour aérosol directement nébulisable sur les pommes de terre et a l'effet antigerminatif de la pomme de terre attendu.

## Revendications

1. Procédé de traitement de fruits et légumes après récolte, comprenant les étapes consistant à
a) préparer une solution aqueuse d'un nitrite de métal alcalin ;
b) dissoudre un produit phytosanitaire contaminé avec des impuretés de type amine primaire aromatique dans un solvant choisi parmi un tensioactif non ionique seul ou en mélange avec un glycol en C₂-C₁₂ de façon à obtenir une solution organique ;
c) mettre en contact les solutions préparées aux étapes précédentes, sous agitation à une température entre 15 et 25°C ;
d) additionner au milieu réactionnel résultant, maintenu sous agitation. à ladite température, un acide minéral fort ;
e) chauffer le milieu réactionnel à une température comprise entre 30 et 70°C ;
f) mélanger avec de l'eau, le milieu réactionnel résultant de l'étape e).
g) traiter les fruits et légumes avec la composition traitante résultant de l'étape f).

2. Procédé selon la revendication 1, **caractérisé en ce que** les impuretés de type amine primaire aromatique ont pour formule
Ar-NH₂ I
dans laquelle Ar représente un noyau aromatique monocyclique ou polycyclique éventuellement substitué par un ou plusieurs radicaux choisis parmi un atome d'halogène; un groupe (C₁-C₈)alkyle; (C₁-C₈)alcoxy; et amino.

3. Procédé selon l'une quelconque des revendications précédentes, **caractérisé en ce que** le produit phytosanitaire est de la diphénylamine contaminée par des impuretés choisis parmi l'aniline, le 2-aminobiphényle, le 4-aminobiphényle et leurs mélanges.

4. Procédé selon l'une quelconque des revendications 1 à 2, **caractérisé en ce que** le produit phytosanitaire est du chlorprofam contaminé par de la métachloroaniline.

5. Procédé selon l'une quelconque des revendications 1 à 2, **caractérisé en ce que** le produit phytosanitaire est de l'éthoxyquin contaminée par de la p-phénétidine.

6. Procédé selon l'une quelconque des revendications précédentes, **caractérisé en ce que** ledit produit phytosanitaire comprend jusqu'à 10 000 ppm en poids d'impuretés de type amine primaire aromatique , de préférence jusqu'à 1000 ppm.

7. Procédé selon l'une quelconque des revendications précédentes, **caractérisé en ce qu'**à l'étape e) le milieu réactionnel est chauffé à une température comprise entre 40 et 50°C.

8. Procédé selon l'une quelconque des revendications précédentes, **caractérisé en ce que** le tensioactif non-ionique est choisi parmi le produit de condensation d'un alcool gras aliphatique en C₈-C₂₂ avec un oxyde d'alkylène en C₂-C₃; la produit de condensation d'un alkylphénol dans lequel la partie alkyle est en C₈-C₂₂ avec un oxyde d'alkylène en C₂-C₃; le produit de condensation d'un acide gras en C₈-C₂₂ avec un oxyde d'alkylène en C₂-C₃; et leurs mélanges.

9. Procédé selon l'une quelconque des revendications précédentes, **caractérisé en ce que** ledit tensioactif a une constante HLB (équilibre hydrophile-lipophile) comprise entre 6 et 18.

10. Procédé selon l'une quelconque des revendications précédentes, **caractérisé en ce que** le glycol en C₂-C₁₂ est choisi parmi l'éthylèneglycol et le propylèneglycol.

11. Procédé selon l'une quelconque des revendications précédentes, **caractérisé en ce que** ladite solution organique du produit phytosanitaire comprend de 5 à 50% en poids dudit produit phytosanitaire.

12. Procédé selon l'une quelconque des revendications précédentes, **caractérisé en ce que** ledit acide minéral fort est choisi : parmi l'acide chlorhydrique et l'acide sulfurique.

13. Procédé selon l'une quelconque des revendications précédentes,. **caractérisé en ce que** le solvant utilisé à l'étape b) est un mélange d'un ou plusieurs tensioactifs non-ioniques.

14. Procédé selon l'une quelconque des revendications précédentes, **caractérisé en ce que** ladite solution organique comprend en outre un ou plusieurs co-solvants choisis parmi les alcools aliphatiques en C₁-C₁₂.

15. Procédé selon l'une quelconque des revendications précédentes, **caractérisé en ce que** ladite solution organique du produit phytosanitaire est constituée de 5 à 50% en poids dudit produit phytosanitaire, de 0 à 80% en poids de tensioactifs non-ioniques, de 0 à 80% en poids de glycols en C₂-C₁₂ et de 0 à 20% en poids d'alcools aliphatiques en C₁-C₁₂.

16. Procédé selon l'une quelconque des revendications précédentes, **caractérisé en ce que** la composition phytosanitaire traitante est obtenue par mise en dispersion dudit milieu réactionnel dans de l'eau, le rapport volumique du milieu réactionnel à l'eau étant compris entre 1/50 et 1/1000.

17. Procédé selon l'une quelconque des revendications précédentes, **caractérisé en ce que** la composition phytosanitaire traitante est obtenue par addition au milieu réactionnel de jusqu'à 30% en poids d'eau.

18. Procédé selon l'une quelconque des revendications précédentes, **caractérisé en ce que**, à l'étape b) la solution organique est obtenue par dissolution du produit phytosanitaire dans un mélange d'un ou plusieurs tensioactifs non ioniques et d'un ou plusieurs glycols.

19. Procédé selon l'une quelconque des revendications précédentes, **caractérisé en ce que**, à l'étape b) la solution organique est obtenue par dissolution du produit phytosanitaire dans un mélange d'un ou plusieurs tensioactifs non ioniques et d'un ou plusieurs cosolvants choisis parmi les alcools aliphatiques en C₁-C₁₂.

## Claims

1. Process for the post-harvest treatment of fruits and vegetables, comprising the stages consisting of
a) preparing an aqueous solution of an alkali metal nitrite;
b) dissolving a phytosanitary product contaminated with impurities of the primary aromatic amine type in a solvent chosen from a nonionic surfactant, by itself or in a mixture with a C₂-C₁₂ glycol, such that an organic solution is obtained;
c) bringing the solutions prepared in the preceding stages into contact at a temperature of between 15 and 25°C, while stirring;
d) adding a strong mineral acid to the resulting reaction mixture, while still stirring at the said temperature;
e) heating the reaction mixture to a temperature of between 30 and 70°C;
f) mixing the reaction mixture resulting from stage e) with water;
g) treating the fruits and vegetables with the treatment composition resulting from stage f).

2. Process according to claim 1, **characterized in that** the impurities of the primary aromatic amine type have the formula
Ar-NH₂ I
in which Ar represents a monocyclic or polycyclic aromatic nucleus optionally substituted by one or more radicals chosen from a halogen atom; and a (C₁-C₈)alkyl; (C₁-C₈)alkoxy; and amino group.

3. Process according to any one of the preceding claims, **characterized in that** the phytosanitary product is diphenylamine contaminated with impurities chosen from aniline, 2-aminobiphenyl, 4-aminobiphenyl and their mixtures.

4. Process according to any one of claims 1 to 2, **characterized in that** the phytosanitary product is chlorpropham contaminated with metachloroaniline.

5. Process according to any one of claims 1 to 2, **characterized in that** the phytosanitary product is ethoxyquin contaminated with p-phenetidine.

6. Process according to any one of the preceding claims, **characterized in that** the said phytosanitary product comprises up to 10,000 ppm by weight of impurities of the primary aromatic amine type, preferably up to 1,000 ppm.

7. Process according to any one of the preceding claims, **characterized in that** in stage e) the reaction mixture is heated to a temperature of between 40 and 50°C.

8. Process according to any one of the preceding claims, **characterized in that** the nonionic surfactant is chosen from the condensation product of a C₈-C₂₂ fatty aliphatic alcohol with a C₂-C₃-alkylene oxide; the condensation product of an alkylphenol in which the alkyl part is C₈-C₂₂ with a C₂-C₃-alkylene oxide; the condensation product of a C₈-C₂₂ fatty acid with a C₂-C₃-alkylene oxide; and their mixtures.

9. Process according to any one of the preceding claims, **characterized in that** the said surfactant has an HLB (hydrophilic-lipophilic balance) constant of between 6 and 18.

10. Process according to any one of the preceding claims, **characterized in that** the C₂-C₁₂ glycol is chosen from ethylene glycol and propylene glycol.

11. Process according to any one of the preceding claims, **characterized in that** the said organic solution of the phytosanitary product comprises 5 to 50% by weight of the said phytosanitary product.

12. Process according to any one of the preceding claims, **characterized in that** the said strong mineral acid is chosen from hydrochloric acid and sulphuric acid.

13. Process according to any one of the preceding claims, **characterized in that** the solvent used in stage b) is a mixture or one or more nonionic surfactants.

14. Process according to any one of the preceding claims, **characterized in that** the said organic solution also comprises one or more co-solvents chosen from C₁-C₁₂ aliphatic alcohols.

15. Process according to any one of the preceding claims, **characterized in that** the said organic solution of the phytosanitary product is made up of 5 to 50% by weight of the said phytosanitary product, 0 to 80% by weight of nonionic surfactants, 0 to 80% by weight of C₂-C₁₂ glycols and 0 to 20% by weight of C₁-C₁₂ aliphatic alcohols.

16. Process according to any one of the preceding claims, **characterized in that** the phytosanitary treatment composition is obtained by dispersing the said reaction mixture in water, the volume ratio of reaction mixture to water being between 1/50 and 1/1,000.

17. Process according to any one of the preceding claims, **characterized in that** the phytosanitary treatment composition is obtained by addition of up to 30% by weight of water to the reaction mixture.

18. Process according to any one of the preceding claims, **characterized in that** in stage b) the organic solution is obtained by dissolving the phytosanitary product in a mixture of one or more nonionic surfactants and one or more glycols.

19. Process according to any one of the preceding claims, **characterized in that** in stage b) the organic solution is obtained by dissolving the phytosanitary product in a mixture of one or more nonionic surfactants and one or more co-solvents chosen from C₁-C₁₂ aliphatic alcohols.

## Patentansprüche

1. Verfahren zur Behandlung von Obst und Gemüse nach der Ernte umfassend die Schritte bestehend aus:
a) Herstellen einer wässrigen Lösung eines Alkalimetall-Nitrits;
b) Auflösen eines mit Verunreinigungen vom Typ primärer, aromatischer Amine verunreinigten phytosanitären Produkts in einem Lösungsmittel, das aus einem nichtionischen, grenzflächenaktiven Stoff, allein oder in Mischung mit einem Glykol aus C₂-C₁₂ ausgewählt ist, zum Erhalten einer organischen Lösung;
c) Herstellen eines Kontakts zwischen den in den vorhergehenden Schritten hergestellten Lösungen unter Rühren bei einer Temperatur zwischen 15 und 25°C;
d) Beimischen einer starken mineralischen Säure zu dem erhaltenen reaktiven Milieu, das unter Rühren auf der Temperatur gehalten wird,;
e) Erwärmen des reaktiven Milieus auf eine Temperatur zwischen 30 und 70°C;
f) Mischen des aus dem Schritt e) erhaltenen reaktiven Milieus mit Wasser;
g) Behandeln des Obsts und Gemüses mit der aus dem Schritt f) erhaltenen Behandlungszusammensetzung.

2. Verfahren nach dem Anspruch 1, **dadurch gekennzeichnet, dass** die Verunreinigungen vom Typ primärer, aromatischer Amine die Formel
Ar-NH₂
aufweisen, in der Ar einen aromatischen, monozyklischen oder polyzyklischen Kern darstellt, der möglicherweise substituiert ist durch ein oder mehrere Radikale, die aus einem Halogenatom, einer (C₁-C₈) Alkylgruppe, (C₁-C₈) Alkoxygruppe und Aminogruppe ausgewählt sind.

3. Verfahren nach einem der vorhergehenden Ansprüche, **dadurch gekennzeichnet, dass** das phytosanitäre Produkt Diphenylamin ist, das mit Verunreinigungen, die aus Anilin, 2-Aminobiphenyl, 4-Aminobiphenyl und deren Mischungen ausgewählt worden sind, verunreinigt ist.

4. Verfahren nach einem der Ansprüche 1 und 2, **dadurch gekennzeichnet, dass** das phytosanitäre Produkt Chlorprofam ist, das mit Metachloranilin verunreinigt ist.

5. Verfahren nach einem der Ansprüche 1 und 2, **dadurch gekennzeichnet, dass** das phytosanitäre Produkt Ethoxycin ist, das mit p-Phenetidin verunreinigt ist.

6. Verfahren nach einem der vorhergehenden Ansprüche, **dadurch gekennzeichnet, dass** das phytosanitäre Produkt bis zu 10000 Gew.-ppm Verunreinigungen vom Typ primärer aromatischer Amine, vorzugsweise bis zu 1000 ppm umfaßt.

7. Verfahren nach einem der vorhergehenden Ansprüche, **dadurch gekennzeichnet, dass** in dem Schritt e) das reaktive Milieu auf eine Temperatur zwischen 40 und 50°C erwärmt wird.

8. Verfahren nach einem der vorhergehenden Ansprüche, **dadurch gekennzeichnet, dass** der nichtionische grenzflächenaktive Stoff aus dem Kondensationsprodukt eines fettigen, aliphatischen C₈-C₂₂ Alkohols mit einem C₂-C₃ Alkylenoxid; dem Kondensationsprodukt eines Alkylphenols, bei dem das Alkylteil aus C₈-C₂₂ mit einem C₂-C₃ Alkylenoxid besteht; dem Kondensationsprodukt einer C₈-C₂₂ Fettsäure mit einem C₂-C₃ Alkylenoxid und deren Mischungen ausgewählt ist.

9. Verfahren nach einem der vorhergehenden Ansprüche, **dadurch gekennzeichnet, dass** der grenzflächenaktive Stoff eine zwischen 6 und 18 liegende HLB-Konstante (hydrophil-lipophiles Gleichgewicht) aufweist.

10. Verfahren nach einem der vorhergehenden Ansprüche, **dadurch gekennzeichnet, dass** das C₂-C₁₂ Glykol aus Ethylenglycol und Propylenglycol ausgewählt ist.

11. Verfahren nach einem der vorhergehenden Ansprüche, **dadurch gekennzeichnet, dass** die organische Lösung des phytosanitären Produkts 5 bis 50 Gew-% des phytosanitären Produkts enthält.

12. Verfahren nach einem der vorhergehenden Ansprüche, **dadurch gekennzeichnet, dass** die starke mineralische Säure aus Chlorwasserstoffsäure und Schwefelsäure ausgewählt ist.

13. Verfahren nach einem der vorhergehenden Ansprüche, **dadurch gekennzeichnet, dass** das in dem Schritt b) benutzte Lösungsmittel eine Mischung aus einem oder mehreren nicht-ionischen grenzflächenaktiven Stoffen ist.

14. Verfahren nach einem der vorhergehenden Ansprüche, **dadurch gekennzeichnet, dass** die organische Lösung weiterhin ein oder mehrere Zusatzlösungsmittel enthält, die aus den aliphatischen C₁-C₁₂ Alkoholen mit ausgewählt sind.

15. Verfahren nach einem der vorhergehenden Ansprüche, **dadurch gekennzeichnet, dass** die organische Lösung des phytosanitären Produkts aus 5 bis 50 Gew-% des phytosanitären Produkts, aus 0 bis 80 Gew-% des nicht-ionischen grenzflächenaktiven Stoffs, aus 0 bis 80 Gew-% C₂-C₁₂ Glykol und aus 0 bis 20 Gew-% aliphatischen C₁-C₁₂ Alkohol zusammengesetzt ist.

16. Verfahren nach einem der vorhergehenden Ansprüche, **dadurch gekennzeichnet, dass** die phytosanitäre Behandlungszusammensetzung durch Dispergieren des reaktiven Milieus in Wasser erhalten wird, wobei das Volumenverhältnis des reaktiven Milieus mit dem Wasser zwischen 1/50 und 1/1000 liegt.

17. Verfahren nach einem der vorhergehenden Ansprüche, **dadurch gekennzeichnet, dass** die phytosanitäre Behandlungszusammensetzung erhalten wird durch Hinzufügen von bis zu 30 Gew-% Wasser zu dem reaktiven Milieu.

18. Verfahren nach einem der vorhergehenden Ansprüche, **dadurch gekennzeichnet, dass** in dem Schritt b) die organische Lösung durch Auflösen des phytosanitären Produkts in einem Gemisch aus einem oder mehreren nicht-ionischen grenzflächenaktivenen Stoffen und einem oder mehreren Glykolen erhalten wird.

19. Verfahren nach einem der vorhergehenden Ansprüche, **dadurch gekennzeichnet, dass** in dem Schritt b) die organische Lösung durch Auflösen des phytosanitären Produkts in einer Mischung aus einem oder mehreren nicht-ionischen, grenzflächenaktiven Stoffen und aus einem oder mehreren Zusatzlösungsmitteln, die aus den aliphatischen C₁-C₁₂ Alkoholen ausgewählt sind, erhalten wird.
